(19) Europäisches Patentamt
European Patent Office
Office européen des brevets

(11) EP 3 958 335 A1

(12) EUROPEAN PATENT APPLICATION
published in accordance with Art. 153(4) EPC

(43) Date of publication:
**23.02.2022 Bulletin 2022/08**

(21) Application number: **20790908.6**

(22) Date of filing: **06.04.2020**

(51) International Patent Classification (IPC):
***H01L 51/30*** (2006.01) ***C07D 307/79*** (2006.01)
***C07D 471/04*** (2006.01) ***H01L 21/336*** (2006.01)
***H01L 29/786*** (2006.01) ***H01L 51/05*** (2006.01)
***H01L 51/40*** (2006.01)

(52) Cooperative Patent Classification (CPC):
**H01L 51/0053; C07D 307/79; C09D 11/033; C09D 11/36; C09D 11/52;** H01L 51/0005; H01L 51/0558

(86) International application number:
**PCT/JP2020/015555**

(87) International publication number:
**WO 2020/213460 (22.10.2020 Gazette 2020/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.04.2019 JP 2019077362**

(71) Applicant: **Daicel Corporation**
**Osaka 530-0011 (JP)**

(72) Inventors:
- **AKAI, Yasuyuki**
  **Tokyo 108-8230 (JP)**
- **YOKOO, Takeshi**
  **Tokyo 108-8230 (JP)**
- **HISHIDA, Masahiro**
  **Tokyo 108-8230 (JP)**

(74) Representative: **Grünecker Patent- und Rechtsanwälte PartG mbB**
**Leopoldstraße 4**
**80802 München (DE)**

# (54) INK COMPOSITION FOR PRODUCING ORGANIC SEMICONDUCTOR DEVICE

(57) Provided is an ink composition for manufacturing an organic semiconductor device, the ink composition allowing an organic semiconductor material with a rigid main chain skeleton to be formed into an ink having a solute concentration that is optimal for a single-crystal formation process. The ink composition for manufacturing an organic semiconductor device contains at least one solvent selected from 2,3-dihydrobenzofuran Compound (A) described below, and at least one type of solute: 2,3-dihydrobenzofuran Compound (A), which includes compounds represented by Formula (a): where in Formula (a), $R^1$, $R^2$, $R^3$, $R^4$, $R^5$ and $R^6$ are as defined in the specification.

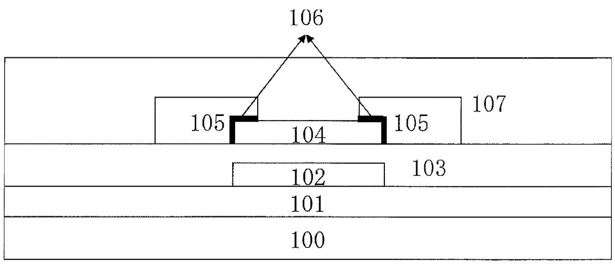


FIG. 1

EP 3 958 335 A1

**Description**

Technical Field

The present disclosure relates to an ink composition for manufacturing an organic semiconductor device. More specifically, the present invention relates to an ink composition used for manufacturing an organic semiconductor device by a coating film forming method including a printing method. The present application claims priority from the Japanese Patent Application No. 2019-077362, filed in Japan on April 15, 2019, the contents of which are incorporated herein by reference.

Background Art

In recent years, organic semiconductors, oxide semiconductors, micro crystal silicon semiconductors, solution-coatable low temperature polysilicon semiconductors, and the like have been studied and developed as candidates for next-generation thin-film active elements. Furthermore, in an effort to prepare for an early stage market launch of flexible substrate devices, in recent years there have been many developments of Flexible Hybrid Electronics (FHE), which combine printed electronics technologies that use organic electronic materials with features such as existing semiconductor and MEMS technologies.

Organic semiconductors, which have been expected to have a central role in printed electronics technologies, have superior characteristics including high mechanical strength against bending and the ability to be formed by a low temperature process and coating method. Therefore, in comparison to other semiconductor materials, organic semiconductors are advantageous in the manufacturing of elements using flexible substrates, and research and development of organic semiconductor materials and devices for practical applications are being actively carried out. In particular, organic single-crystal semiconductor films that enable high performance organic semiconductor devices are attracting attention. Coating methods such as an edge-casting method and a continuous edge-casting method have been proposed as techniques for manufacturing such organic single-crystal semiconductor films (see Patent Document 1).

In general, it is known that with n-type organic semiconductor materials, it is more difficult to produce crystalline thin films than with p-type organic semiconductor materials, and it is difficult to obtain high transistor characteristics (e.g., carrier mobility). Gas phase growth processes (such as a vapor deposition method) that use large and expensive equipment such as ultra-high vacuum devices have been used for single crystallization and thin film formation of n-type organic semiconductor materials. However, with film deposition methods that use ultra-high vacuum devices, there is a limit to how much the surface area can be expanded, and the manufacturing process itself is very expensive.

For this reason, there is a demand for a composition that contains an n-type organic semiconductor material and can be produced by a coating method. For example, Patent Document 2 describes a composition for an organic thin-film transistor prepared by dissolving an organic compound having a perylene bisimide skeleton, such as a perylene diimide (PDI), in an organic solvent (anisole). The carrier mobility of the organic thin-film transistor manufactured by applying the composition described above is reported to be $1 \times 10^{-4}$ $cm^2/Vs$ or greater.

Citation List

Patent Documents

Patent Document 1: JP 2015-185620 A

Patent Document 2: JP 2018-006745 A

Summary of Invention

Technical Problem

However, the transistor characteristics of an organic thin-film transistor obtained using the organic thin-film transistor composition described in Patent Document 2 are not at a sufficiently satisfactory level. As the reason for this, it is speculated that because a low molecular weight n-type organic semiconductor material, such as one having a perylene bisimide skeleton, has a condensed ring skeleton with highly conjugated π-electrons, and has a rigid skeletal structure of the main chain, solubility in a solvent is extremely low, and it is difficult to produce a good quality organic single-crystal thin film. That is, with known compositions for organic thin-film transistors, it cannot be said that the blended

n-type organic semiconductor material is formed into an ink at concentration optimal for a single crystal formation process.

In light of such circumstances, an object of the present disclosure is to provide an ink composition for manufacturing an organic semiconductor device, the ink composition allowing an n-type organic semiconductor material having a rigid main chain skeleton to be formed into an ink having a solute concentration optimal for a single crystal formation process.

Solution to Problem

As a result of diligent research to solve the problems described above, the inventors of the present disclosure found that the solubility of an n-type organic semiconductor material having a rigid main chain skeleton is high in a specific benzofuran compound, and such benzofuran compound is suitable as a solvent that allows the organic semiconductor material to be formed into an ink having a solute concentration optimal for a single-crystal formation process. The invention according to the present disclosure was completed based on these findings.

That is, the invention according to the present disclosure provides an ink composition for manufacturing an organic semiconductor device, the ink composition containing at least one solvent selected from 2,3-dihydrobenzofuran Compound (A) described below, and at least one type of solute:

2,3-dihydrobenzofuran Compound (A), which includes compounds represented by Formula (a):

[Chem. 1]

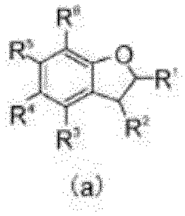

(a)

where in Formula (a), $R^1$, $R^2$, $R^3$, $R^4$, $R^5$, and $R^6$ each represent a hydrogen atom, a halogen atom, a $C_{1-20}$ alkyl group optionally having a substituent selected from Group 1, a $C_{2-22}$ alkenyl group optionally having a substituent selected from Group 1, a $C_{2-22}$ alkynyl group optionally having a substituent selected from Group 1, a $C_{1-20}$ alkoxy group optionally having a substituent selected from Group 1, a $C_{1-20}$ alkylthio group optionally having a substituent selected from Group 1, a $C_{2-20}$ alkylcarbonyl group optionally having a substituent selected from Group 1, a $C_{2-20}$ alkoxycarbonyl group optionally having a substituent selected from Group 1, a di- or mono- $C_{1-20}$ alkylamino group optionally having a substituent selected from Group 1, a $C_{6-20}$ aryl group optionally having a substituent selected from Group 2, a monovalent heterocyclic group optionally having a substituent selected from Group 2, or a $C_{3-20}$ cycloalkyl group optionally having a substituent selected from Group 2.

Group 1 mentioned above includes a halogen atom, a sulfonyl group, a hydroxy group, an aldehyde group (-CHO), a carbonyl group, a carboxyl group, a nitro group, an amino group, a sulfo group ($-SO_3H$), an ether group, a $C_{1-20}$ alkylthio group, a di- or mono- $C_{1-20}$ alkylamino group, a $C_{6-20}$ aryl group, a monovalent heterocyclic group, and $C_{3-20}$ substituted silyl group.

Group 2 mentioned above includes a substituent selected from Group 1, a $C_{1-20}$ alkyl group optionally having a substituent selected from Group 1, a $C_{2-20}$ alkenyl group optionally having a substituent selected from Group 1, and a $C_{2-20}$ alkynyl group optionally having a substituent selected from Group 1.

In the aforementioned ink composition for manufacturing an organic semiconductor device, $R^1$, $R^2$, $R^3$, $R^4$, $R^5$, and $R^6$ each preferably represent a hydrogen atom, a halogen atom or a $C_{1-20}$ alkyl group optionally having a substituent selected from Group 1.

In the ink composition for manufacturing an organic semiconductor device described above, 2,3-dihydrobenzofuran Compound (A) is preferably 2,3-dihydrobenzofuran.

In the aforementioned ink composition for manufacturing an organic semiconductor device, the solute may be an n-type organic semiconductor material.

The aforementioned ink composition for manufacturing an organic semiconductor device may further include a macromolecular compound as a second component in addition to the solute.

The aforementioned ink composition for manufacturing an organic semiconductor device may be used for producing an organic single-crystal semiconductor film by a drop-casting method, an inkjet printing method, an edge-casting method, or a continuous edge-casting method.

Advantageous Effects of Invention

Because the ink composition for manufacturing an organic semiconductor device according to an embodiment of the present disclosure has the structure described above, an ink concentration exhibiting excellent n-type semiconductor characteristics can be achieved even when an n-type organic semiconductor material having a rigid main chain skeleton is used. Additionally, according to the ink composition for manufacturing an organic semiconductor device of the present disclosure, a high performance organic single-crystal semiconductor element can be efficiently formed in a temperature range in which a film can be formed through coating, and a highly reliable organic single-crystal semiconductor film can be efficiently formed at a low cost. Furthermore, the ink composition for manufacturing an organic semiconductor device of the present disclosure can be applied through a coating method, and therefore, an organic single-crystal semiconductor film having high uniformity in a large surface area can be formed. Therefore, a flexible device with unprecedentedly high-performance can be provided with high efficiency and at a low cost through an organic thin-film transistor.

Specifically, by forming an organic single-crystal semiconductor by a drop-casting method, an inkjet printing method, an edge-casting method, or a continuous edge-casting method using the ink composition for manufacturing an organic semiconductor device according to an embodiment of the present disclosure, a flexible device equipped with a high-performance organic CMOS circuit that combines a p-type transistor having a field effect mobility $\mu$p of 10 [$cm^2$/Vs] or greater and an n-type transistor having a field effect mobility $\mu$n of 0.1 [$cm^2$/Vs] or greater can be provided.

Brief Description of Drawings

FIG. 1 is a schematic diagram of a cross-sectional structure of an example of an organic thin-film transistor.
FIG. 2 is a drawing illustrating the concept of an example of a continuous edge-casting method.

Description of Embodiments

Ink composition for manufacturing organic semiconductor device

An ink composition for manufacturing an organic semiconductor device according to an embodiment of the present disclosure (hereinafter may simply be referred to as the "ink composition according to an embodiment of the present disclosure") contains at least one solvent selected from 2,3-dihydrobenzofuran Compound (A) described below, and at least one solute.

2,3-dihydrobenzofuran Compound (A), including compounds represented by Formula (a) below:

[Chem. 2]

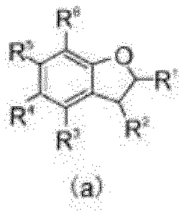

(a)

In Formula (a), $R^1$, $R^2$, $R^3$, $R^4$, $R^5$, and $R^6$ each represent a hydrogen atom, a halogen atom, a $C_{1-20}$ alkyl group optionally having a substituent selected from Group 1, a $C_{2-22}$ alkenyl group optionally having a substituent selected from Group 1, a $C_{2-22}$ alkynyl group optionally having a substituent selected from Group 1, a $C_{1-20}$ alkoxy group optionally having a substituent selected from Group 1, a $C_{1-20}$ alkylthio group optionally having a substituent selected from Group 1, a $C_{2-20}$ alkylcarbonyl group optionally having a substituent selected from Group 1, a $C_{2-20}$ alkoxycarbonyl group optionally having a substituent selected from Group 1, a di- or mono- $C_{1-20}$ alkylamino group optionally having a substituent selected from Group 1, a $C_{6-20}$ aryl group optionally having a substituent selected from Group 2, a monovalent heterocyclic group optionally having a substituent selected from Group 2, or a $C_{3-20}$ cycloalkyl group optionally having a substituent selected from Group.

Group 1 mentioned above includes a halogen atom, a sulfonyl group, a hydroxy group, an aldehyde group (-CHO), a carbonyl group, a carboxyl group, a nitro group, an amino group, a sulfo group (-$SO_3$H), an ether group, a $C_{1-20}$ alkylthio group, a di- or mono-$C_{1-20}$ alkylamino group, a $C_{6-20}$ aryl group, a monovalent heterocyclic group, and a

$C_{3-20}$ substituted silyl group.

Group 2 mentioned above includes a substituent selected from Group 1, a $C_{1-20}$ alkyl group optionally having a substituent selected from Group 1, a $C_{2-20}$ alkenyl group optionally having a substituent selected from Group 1, and a $C_{2-20}$ alkynyl group optionally having a substituent selected from Group 1.

[2,3-dihydrobenzofuran Compound (A)]

The ink composition according to an embodiment of the present disclosure contains, as a solvent, at least one solvent selected from 2,3-dihydrobenzofuran Compound (A), which includes compounds represented by Formula (a). The solubility of even an n-type organic semiconductor material having a rigid main chain skeleton is high in 2,3-dihydrobenzofuran Compound (A), and thus 2,3-dihydrobenzofuran Compound (A) is suitable as a solvent that allows the organic semiconductor material to be formed into an ink having a solute concentration that is optimal for a single-crystal formation process. The ink composition according to an embodiment of the present disclosure may contain only one compound from 2,3-dihydrobenzofuran Compound (A), or may contain two or more compounds from 2,3-benzofuran Compound (A).

In Formula (a), $R^1$, $R^2$, $R^3$, $R^4$, $R^5$, and $R^6$ each represent a hydrogen atom, a halogen atom, a $C_{1-20}$ alkyl group optionally having a substituent selected from Group 1, a $C_{2-22}$ alkenyl group optionally having a substituent selected from Group 1, a $C_{2-22}$ alkynyl group optionally having a substituent selected from Group 1, a $C_{1-20}$ alkoxy group optionally having a substituent selected from Group 1, a $C_{1-20}$ alkylthio group optionally having a substituent selected from Group 1, a $C_{2-20}$ alkylcarbonyl group optionally having a substituent selected from Group 1, a $C_{2-20}$ alkoxycarbonyl group optionally having a substituent selected from Group 1, a di- or mono- $C_{1-20}$ alkylamino group optionally having a substituent selected from Group 1, a $C_{6-20}$ aryl group optionally having a substituent selected from Group 2, a monovalent heterocyclic group optionally having a substituent selected from Group 2, or a $C_{3-20}$ cycloalkyl group optionally having a substituent selected from Group 2 above.

Examples of the halogen atoms described above include a fluorine atom, a chlorine atom, a bromine atom, and an iodine atom.

Examples of the $C_{1-20}$ alkyl group include linear or branched-chain alkyl groups having from 1 to 20 carbons, such as a methyl group, an ethyl group, an n-propyl group, an isopropyl group, an n-butyl group, an isobutyl group, a sec-butyl group, a tert-butyl group, an n-pentyl group, an isopentyl group, a neopentyl group, a tert-pentyl group, an n-hexyl group, an isohexyl group, a heptyl group, an octyl group, an nonyl group, and a decyl group.

Examples of the $C_{2-22}$ alkenyl group include linear or branched-chain alkenyl groups having from 2 to 22 carbons, such as vinyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl, 1-heptenyl, 1-octenyl, 1-nonenyl, and 1-decenyl.

Examples of the $C_{2-22}$ alkynyl group include linear or branched-chain alkynyl groups having from 2 to 22 carbons, such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-heptynyl, 1-octynyl, 1-nonynyl, and 1-decynyl.

Examples of the $C_{1-20}$ alkoxy group include linear or branched-chain alkoxy groups having from 1 to 20 carbons, such as a methoxy group, an ethoxy group, an n-propoxy group, an isopropoxy group, an n-butoxy group, an isobutoxy group, a sec-butoxy group, a tert-butoxy group, an n-pentyloxy group, an isopentyloxy group, a neopentyloxy group, a tert-pentyloxy group, an n-hexyloxy group, an isohexyloxy group, a heptyloxy group, an octyloxy group, a nonyloxy group, and a decyloxy group.

Examples of the $C_{1-20}$ alkylthio group include linear or branched-chain alkylthio groups having from 1 to 20 carbons, such as a methylthio group, an ethylthio group, an n-propylthio group, an isopropylthio group, an n-butylthio group, an isobutylthio group, a sec-butylthio group, a tert-butylthio group, an n-pentylthio group, an isopentylthio group, a neopentylthio group, a tert-pentylthio group, an n-hexylthio group, an isohexylthio group, a heptylthio group, an octylthio group, a nonylthio group, and a decylthio group.

Examples of the $C_{2-20}$ alkylcarbonyl group include linear or branched-chain alkylcarbonyl groups having from 2 to 20 carbons, such as an acetyl group, a propionyl group, an n-butyryl group, an isobutyryl group, an n-butylcarbonyl group, an isobutylcarbonyl group, a sec-butylcarbonyl group, a tert-butylcarbonyl group, an n-pentylcarbonyl group, an isopentylcarbonyl group, a neopentylcarbonyl group, a tert-pentylcarbonyl group, an n-hexylcarbonyl group, an isohexylcarbonyl group, a heptylcarbonyl group, an octylcarbonyl group, a nonylcarbonyl group, and a decylcarbonyl group.

Examples of the $C_{2-20}$ alkoxycarbonyl group include linear or branched-chain alkoxycarbonyl groups having from 2 to 20 carbons, such as a methoxycarbonyl group, an ethoxycarbonyl group, an n-propoxycarbonyl group, an isopropoxycarbonyl group, an n-butoxycarbonyl group, an isobutoxycarbonyl group, a sec-butoxycarbonyl group, a tert-butoxycarbonyl group, an n-pentyloxycarbonyl group, an isopentyloxycarbonyl group, a neopentyloxycarbonyl group, a tert-pentyloxycarbonyl group, an n-hexyloxycarbonyl group, an isohexyloxycarbonyl group, an heptyloxycarbonyl group, an octyloxycarbonyl group, a nonyloxycarbonyl group, and a decyloxycarbonyl group.

Examples of the di- or mono- $C_{1-20}$ alkylamino group include amino groups mono- or di-substituted with a $C_{1-20}$ alkyl group mentioned above, such as a methylamino group, an ethylamino group, a propylamino group, an isopropylamino group, a butylamino group, an isobutylamino group, a tert-butylamino group, an N,N-dimethylamino group, an N,N-diethylamino group, an N,N-dipropylamino group, an N,N-diisopropylamino group, an N,N-dibutylamino group, an N,N-diisobutylamino group, an N,N-ditert-butylamino group, and an N-methyl-N-ethylamino group.

Examples of the $C_{6-20}$ aryl group include aryl groups having from 6 to 20 carbons, such as a phenyl group, a naphthyl group, an anthryl group, a phenanthryl group, an acenaphthylenyl group, and a biphenylyl group.

Examples of the monovalent heterocyclic group include from 5- to 22-membered (preferably 5- or 6-membered) aromatic heterocyclic groups and non-aromatic heterocyclic groups having in the ring a carbon atom and from 1 to 4 heteroatoms selected from an oxygen atom, a sulfur atom and a nitrogen atom.

Examples of the aromatic heterocyclic group include monocyclic aromatic heterocyclic groups, such as a furyl group, a thienyl group, a pyridyl group, a pyrrolyl group, an imidazolyl group, a pyrazolyl group, and a thiazolyl group; and fused aromatic heterocyclic groups such as a quinolyl group and isoquinolyl.

Examples of the non-aromatic heterocyclic group include monocyclic non-aromatic heterocyclic groups, such as a piperidyl group, a morpholinyl group, a piperazinyl group, and a tetrahydrofuryl group; and fused non-aromatic heterocyclic groups, such as a chromenyl group, a tetrahydroquinolinyl group, and a tetrahydroisoquinolinyl group.

Examples of the $C_{3-20}$ cycloalkyl group include cyclic alkyl groups having from 3 to 20 carbons, such as a cyclopropyl group, a cyclobutyl group, a cyclopentyl group, a cyclohexyl group, a cycloheptyl group, a cyclooctyl group, a cyclononyl group, and a cyclodecyl group.

Examples of the sulfonyl group include $C_{1-20}$ alkylsulfonyl groups and $C_{6-20}$ arylsulfonyl groups.

Examples of the $C_{1-20}$ alkylsulfonyl group include linear or branched-chain alkylsulfonyl groups having from 1 to 20 carbons, such as a methylsulfonyl group, an ethyl sulfonyl group, an n-propylsulfonyl group, an isopropylsulfonyl group, an n-butylsulfonyl group, an isobutylsulfonyl group, a sec-butylsulfonyl group, a tert-butyl sulfonyl group, an n-pentylsulfonyl group, an isopentylsulfonyl group, a neopentylsulfonyl group, a tert-pentylsulfonyl group, an n-hexylsulfonyl group, an isohexylsulfonyl group, a heptylsulfonyl group, an octylsulfonyl group, an nonylsulfonyl group, and a decylsulfonyl group.

Examples of the $C_{6-20}$ arylsulfonyl group include arylsulfonyl groups having from 6 to 20 carbons, such as a phenylsulfonyl group, a naphthylsulfonyl group, an anthrylsulfonyl group, a phenanthrylsulfonyl group, an acenaphthylenylsulfonyl group, and a biphenylylsulfonyl group.

Examples of the carbonyl group include the above-mentioned $C_{2-20}$ alkylcarbonyl groups, the above-mentioned $C_{2-20}$ alkoxycarbonyl groups, and $C_{7-20}$ arylcarbonyl groups.

Examples of the $C_{7-20}$ arylcarbonyl group include arylcarbonyl groups having from 7 to 20 carbons, such as a benzoyl group, a naphthylcarbonyl group, an anthrylcarbonyl group, a phenanthrylcarbonyl group, an acenaphthylenylcarbonyl group, and a biphenylylcarbonyl group.

Examples of the ether group include the above-mentioned $C_{1-20}$ alkoxy groups and $C_{6-20}$ aryloxy groups.

Examples of the $C_{6-20}$ aryloxy group include aryloxy groups having from 6 to 20 carbons, such as a phenoxy group, a naphthyloxy group, an anthryloxy group, a phenanthryloxy group, an acenaphthylenyloxy group, and a biphenylyloxy group.

Examples of the $C_{3-20}$ substituted silyl group include silyl groups having from 1 to 3, preferably 3, substituents selected from the above-mentioned $C_{1-20}$ alkyl groups and the above-mentioned $C_{6-20}$ aryl groups, such as a trimethylsilyl group, a triethylsilyl group, a tert-butyldimethylsilyl group, a tert-butyldiphenylsilyl group, and a triisopropylsilyl group.

The $C_{1-20}$ alkyl groups, $C_{2-22}$ alkenyl groups, $C_{2-22}$ alkynyl groups, $C_{1-20}$ alkoxy groups, $C_{1-20}$ alkylthio groups, $C_{2-20}$ alkylcarbonyl groups, $C_{2-20}$ alkoxycarbonyl groups, and di- or mono-$C_{1-20}$ alkylamino groups descried above may have a substituent selected from Group 1, while the $C_{6-20}$ aryl groups, monovalent heterocyclic groups, and $C_{3-20}$ cycloalkyl groups may have a substituent selected from Group 2 above. The number of substituents is not limited, but is preferably from 1 to 3. When two or more of the above substituents are contained, the two or more substituents may be the same or different.

For $R^1$, $R^2$, $R^3$, $R^4$, $R^5$, and $R^6$, each of the substituents may be bonded to each other to further form a ring.

From the perspectives of the solubility of solutes in 2,3-dihydrobenzofuran Compound (A) and the procurement ease of 2,3-dihydrobenzofuran Compound (A), $R^1$, $R^2$, $R^3$, $R^4$, $R^5$ and $R^6$ are each preferably a hydrogen atom, a halogen atom, a $C_{1-20}$ alkyl group optionally having a substituent selected from Group 1, or a $C_{1-20}$ alkoxy group optionally having a substituent selected from Group 1, and are particularly preferably a hydrogen atom or a $C_{1-20}$ alkyl group.

Preferred specific examples of 2,3-dihydrobenzofuran Compound (A) include 2,3-dihydrobenzofuran and 2,3-dihydro-2-methylbenzofuran, and these can be used alone or in a combination of two or more. Among these, 2,3-dihydrobenzofuran is preferable from perspectives such as the solubility of an organic semiconductor material and the film formability of an organic single-crystal semiconductor.

The proportion of 2,3-dihydrobenzofuran Compound (A) in the total solvent is, for example, greater than or equal to 90 wt.%, preferably greater than or equal to 93 wt.%, more preferably greater than or equal to 95 wt.%, and

particularly preferably 100 wt.%. When the proportion of 2,3-dihydrobenzofuran Compound (A) in the entire solvent is greater than or equal to the lower limit described above, the solubility of the organic semiconductor material and the film formability of the organic single-crystal semiconductor are more excellent. Thus, in particular, formation of an n-type organic semiconductor material into an ink at a solution concentration optimum for a single-crystal formation process is further facilitated.

The purity of 2,3-dihydrobenzofuran Compound (A) is, for example, 98.0% or greater, preferably 98.5% or greater, more preferably 99.0% or greater, even more preferably 99.5% or greater, and particularly preferably 99.9% or greater. It is thought that since the proportion of the organic semiconductor compound in the ink composition for an organic semiconductor device is very small, impurities in the solvent greatly affect organic semiconductor single-crystal film formation. Thus, in a case where the purity of 2,3-dihydrobenzofuran Compound (A) is greater than or equal to 98.0%, high-quality, clean film formation is more likely to be obtained. The purity of 2,3-dihydrobenzofuran Compound (A) can be measured through gas chromatography (GC).

When 2,3-dihydrobenzofuran Compound (A) is used as the solvent, the solubility of the organic semiconductor material and the film formability of an organic single-crystal semiconductor are more excellent than a case in which another common solvent is used (e.g., hydrocarbon solvents such as hexane and octane, and ether solvents such as anisole), and while the reason for this is not clear, it is presumed to be as follows. The solvent used in the ink composition for an organic semiconductor device is required to have high solubility, high film formability, and high printing properties. For example, in order for the organic semiconductor device to obtain high carrier mobility, the film thickness of the organic semiconductor single-crystal film must be controlled. Therefore, high solubility is required of the solvent. In addition, because the ink composition for an organic semiconductor device is applied (film-formed) with a printing method, volatility of the solvent during printing affects the film formability. For example, when the solvent is a high boiling point solvent (for example, 200°C or higher), the volatility of the solvent is poor, a high-temperature printing process is required, and issues such as deformation or damage of a plastic base material may occur, and when the solvent is a low boiling point solvent (for example, 100°C or lower), the volatility is high, and thus it is difficult to maintain the concentration of the ink composition for an organic semiconductor device. Therefore, high film formability and high printing properties are required in the solvent. In the course of examining solvents suitable for an ink composition for an organic semiconductor device from amongst numerous solvents, the present inventors newly discovered that 2,3-dihydrobenzofuran Compound (A) possesses a good balance of high solubility, high film formability, and high printing properties, and can superbly exhibit the abovementioned properties. Therefore, it is presumed that 2,3-dihydrobenzofuran Compound (A) exhibits more excellent solubility of organic semiconductor materials and film formability of organic single-crystal semiconductors than other common solvents. However, the above is merely conjecture and does not limit the invention according to the present disclosure.

The ink composition according to an embodiment of the present disclosure may contain a solvent (other solvent) besides 2,3-dihydrobenzofuran Compound (A). The other solvent described above is a solvent that is ordinarily used in electronic material applications, and examples include a solvent that is miscible with 2,3-dihydrobenzofuran Compound (A). One or two other solvents may be contained. The proportion of other solvents in the total solvent is, for example, from 0 wt.% to less than 10 wt.%, preferably from 0 wt.% to less than 7 wt.%, and more preferably from 0 w.% to less than 5 wt.%. Among these proportions, from the perspective of more effectively exhibiting the function of 2,3-dihydrobenzofuran Compound (A) as a solvent, it is particularly preferable that no other solvent is included (0 wt.%).

The water content of the solvent used in the composition according to an embodiment of the present disclosure is preferably not greater than 0.25 wt.%. When the water content in the composition according to an embodiment of the present disclosure is high, the carrier mobility tends to decrease. It is speculated that this is due to crystallization of the organic semiconductor material being inhibited, or moisture being trapped by the carrier. The water content is preferably not greater than 0.19 wt%, and more preferably not greater than 0.05 wt%. Note that the water content can be measured by the Karl Fischer method.

Solute

As the solute contained in the ink composition according to an embodiment of the present disclosure, a known n-type organic semiconductor material can be used without particular limitation.

From the perspective of field effect mobility and the like, the n-type organic semiconductor material used as the solute contained in the ink composition according to an embodiment of the present disclosure is preferably a compound having a rigid main chain skeleton, and examples thereof include compounds represented by Formula (1) below.

[Chem. 3]

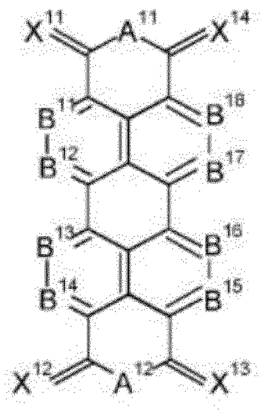

(1)

[In Formula (1), $A^{11}$ and $A^{12}$ each independently represent -O-, -N($R^N$)-, or - P($R^N$)-. $B^{11}$ to $B^{18}$ each independently represent -N= or -C($R^M$)=. $R^N$ and $R^M$ represent a hydrogen atom or a substituent. $X^{11}$ to $X^{14}$ each independently represent an oxygen atom or a sulfur atom.]

In Formula (1), $A^{11}$ and $A^{12}$ each represent -O-, -N($R^N$)-, or -P($R^N$)-. $A^{11}$ and $A^{12}$ are each preferably -N($R^N$)-. $A^{11}$ and $A^{12}$ may be the same as or different from each other, but are preferably the same, and are more preferably both -N($R^N$)-.

$R^N$ represents a hydrogen atom or a substituent. The substituent that can be adopted as $R^N$ is not limited. For example, the substituent can be one selected from the following substituent group Z.

Substituent group Z:

The substituent group Z includes halogen atoms (including a fluorine atom, a chlorine atom, a bromine atom, or a iodine atom; preferably a fluorine atom or a chlorine atom); alkyl groups (including alkyl groups having a substituent, and alicyclic hydrocarbon groups; preferably, an alkyl group having from 1(3) to 40 carbons, more preferably from 1(3) to 20 carbons, and particularly preferably from 4 to 20 carbons, the numbers in parenthesis representing the number of carbons when the alkyl group is a cycloalkyl group; examples of the alkyl group including methyl, ethyl, propyl, 2-methylpropyl, butyl, amyl, pentyl, 2,2-dimethylpropyl, hexyl, 1-methylpentyl [(1S)-1-methylpentyl, (1R)-1-methylpentyl], heptyl, octyl, nonyl, decyl, undecyl, dodecyl, tridecyl, tetradecyl, pentadecyl, 2,6-dimethyloctyl, icosyl, 2-decyltetradecyl, 2-hexyldodecyl, 2-ethyloctyl, 2-decyltetradecyl, 2-butyldecyl, 1-octylnonyl, 2-ethyloctyl, 2-octyldecyl, 2-octyldodecyl, 7-hexylpentadecyl, 2-octyltetradecyl, 2-ethylhexyl, cyclopropyl, cyclobutyl, cyclopentyl, cyclohexyl, adamantyl, benzyl, p-chlorobenzyl, 2-phenylethyl, 3-phenylpropyl, trifluoromethyl, perfluoroethyl, 2,2,3,3,4,4,4-heptafluorobutyl, $C_5F_{11}C_2H_4$-, 3-aminopropyl, 4-aminobutyl, 5-ethoxypentyl, (meth)acryloxypropyl, (meth)acryloxypentyl, 4-hydroxybutyl, 4-sulfobutyl, 10-phosphonodecyl, 2-hydroxyethoxymethyl, 2-imidazolylethoxymethyl, and 4-(N,N-dimethylamino)butyl); alkenyl groups (preferably having from 2 to 20 carbons, more preferably having from 2 to 12 carbons, particularly preferably from 2 to 8 carbons, such as vinyl, allyl, 2-butenyl, 1-pentenyl, and 4-pentenyl); alkynyl groups (preferably having from 2 to 20 carbons, more preferably having from 2 to 12 carbons, particularly preferably having from 2 to 8 carbons, such as propargyl, 1-pentynyl, trimethylsilylethynyl, triethylsilylethynyl, tri-i-propylsilylethynyl, and 2-p-propylphenylethynyl); aryl groups (preferably having from 6 to 20 carbons, more preferably from 6 to 12 carbons, such as phenyl, naphthyl, 2,4,6-trimethylphenyl, p-(t-butyl)phenyl, 4-methyl-2,6-dipropylphenyl, 4-fluorophenyl, 4-trifluoromethylphenyl, p-pentylphenyl, 3,4-dipentylphenyl, p-heptoxyphenyl, and 3,4-diheptoxyphenyl); heterocyclic groups (including as ring-constituting atoms, at least one heteroatom and from 1 to 30 carbon atoms; examples of the heteroatom include a nitrogen atom, an oxygen atom, and a sulfur atom, and the number of heteroatoms is not limited, but is, for example, from 1 to 2; the number of the ring-constituting carbon atoms is preferably from 3 to 20 and more preferably from 3 to 12; the heterocyclic group is preferably a 5- or 6-membered ring or a fused ring thereof; the heterocyclic group includes aromatic heterocyclic groups (heteroaryl groups) and aliphatic heterocyclic groups; examples include, thienyl, thiazolyl, imidazolyl, pyridyl, pyrimidinyl, quinolyl, furanyl, selenophenyl [$C_4H_3Se$], piperidyl, morpholino, benzoxazolyl, benzimidazolyl, benzthiazolyl, 2-hexylfuranyl, and pyranyl); silyl groups (preferably having from 3 to 40 carbons, more preferably from 3 to 30 carbons, and particularly preferably from 3 to 24 carbons, such as trimethylsilyl, triphenylsilyl, and dimethylphenylsilyl); alkoxy groups (preferably having from 1 to 20 carbons, more preferably from 1 to 12 carbons, and particularly preferably from 1 to 8 carbons, such as methoxy, ethoxy, and butoxy); amino groups (preferably having from 0 to 20 carbons, more

preferably from 0 to 10 carbons, and particularly preferably from 0 to 6 carbons, such as amino, methylamino, dimethylamino, diethylamino, dibenzylamino, and anilino); aryloxy groups (preferably having from 6 to 20 carbons, more preferably from 6 to 16 carbons, and particularly preferably from 6 to 12 carbons, such as phenyloxy and 2-naphthyloxy); acyl groups (preferably having from 1 to 20 carbons, more preferably from 1 to 16 carbons, and particularly preferably from 1 to 12 carbons, such as acetyl, hexanoyl, benzoyl, formyl, and pivaloyl); alkoxycarbonyl groups (preferably having from 2 to 20 carbons, more preferably from 2 to 16 carbons, and particularly preferably from 2 to 12 carbons, such as methoxycarbonyl and ethoxycarbonyl); aryloxycarbonyl groups (preferably having from 7 to 20 carbons, more preferably from 7 to 16 carbons, and particularly preferably from 7 to 10 carbons, such as phenyloxycarbonyl); acyloxy groups (preferably having from 2 to 20 carbons, more preferably from 2 to 16 carbons, and particularly preferably from 2 to 10 carbons, such as acetoxy, benzoyloxy or (meth)acryloyloxy); acylamino groups (preferably having from 2 to 20 carbons, more preferably from 2 to 16 carbons, and particularly preferably from 2 to 10 carbons, such as acetylamino and benzoylamino); aminocarbonylamino groups (preferably having from 2 to 20 carbons, more preferably from 2 to 16 carbons, and particularly preferably from 2 to 12 carbons, such as a ureido group); alkoxy or aryloxycarbonylamino groups (preferably having from 2(7) to 20 carbons, more preferably from 2(7) to 16 carbons, and particularly preferably from 2(7) to 12 carbons; the numbers in parenthesis representing the number of carbons in the case of an aryloxycarbonylamino group; examples including methoxycarbonylamino or phenyloxycarbonylamino); alkyl or arylsulfonylamino groups, alkylthio groups (preferably having from 1 to 20 carbons, more preferably from 1 to 16 carbons, and particularly preferably from 1 to 12 carbons, such as methylthio, ethylthio, and octylthio); arylthio groups (preferably having from 6 to 20 carbons, more preferably from 6 to 16 carbons, and particularly preferably from 6 to 12 carbons, such as a phenylthio group); an alkyl or arylsulfinyl group, an alkyl or arylsulfonyl group, a silyloxy group, a heterocyclic oxy group, a carbamoyl group, a carbamoyloxy group, a heterocyclic thio group, a sulfamoyl group, an aryl or heterocyclic azo group, an imide group, a phosphino group, a phosphinyl group, a phosphinyloxy group, a phosphinylamino group, a hydrazino group, an imino group, a cyano group, a hydroxy group, a nitro group, a mercapto group, a sulfo group, a carboxy group, a hydroxamic acid group, a sulfino group, a boronic acid group ($-B(OH)_2$), a phosphato group ($-OPO(OH)_2$), a phosphono group ($-PO(OH)_2$), and a sulfato group ($-OSO_3H$).

A group selected from the abovementioned substituent group Z that can be adopted as $R^N$ is preferably an alkyl group, an aryl group, an alkenyl group, an alkynyl group, a heterocyclic group, or a silyl group, more preferably an alkyl group (preferably having from 1 to 20 carbons), an aryl group (preferably having from 6 to 20 carbons), or a heteroaryl group (containing at least one or more of the above heteroatoms as a ring-constituting atom; preferably a 5- or 6-membered ring or a fused ring thereof; and the number of ring-constituting carbon atoms being preferably from 3 to 20), and even more preferably an alkyl group (particularly preferably having from 4 to 20 carbons).

The group selected from the substituent group Z described above may further include a substituent. Examples of such a substituent include a group selected from the substituent group Z. The number of substituents that may be further contained in the group further containing a substituent (also referred to as a combined group) is not limited, but is preferably, for example, from 1 to 6, and more preferably from 1 to 3.

The combined group is not limited, and examples thereof include a group in which each of the aforementioned groups preferred as a group selected from the substituent group Z described above is substituted with another group selected from the substituent group Z. Specific examples include a halogen atom, an alkyl group, an aryl group, a heterocyclic group (heteroaryl group), an alkoxy group (including a hydroxyalkoxy group, an alkyl halide group, and a heteroarylalkoxy group), an amino group, an acyloxy group, a hydroxy group, an alkyl group having a group selected from the group consisting of a sulfato group and a phosphono group as a substituent, an aryl halide group or (fluorinated) alkylaryl group, and an alkynyl group having a silyl group as a substituent. Furthermore, the examples also include a group in which one hydrogen atom is removed from the compound represented by Formula (1).

More specifically, the examples include the groups in the examples of the substituent group Z described above, and groups in the following exemplified compounds or compounds used in Examples.

Among these, the combined group is preferably an alkyl group having a halogen atom as a substituent (alkyl halide group) or an alkyl group having an aryl group as a substituent, more preferably an alkyl group having a fluorine atom as a substituent (alkyl fluoride group) or an alkyl group having an aryl group as a substituent, particularly preferably an alkyl group having an aryl group as a substituent.

The substituent that can be adopted as $R^N$ is more preferably an (unsubstituted) alkyl group, an alkyl halide group, or an alkyl group having an aryl group as a substituent.

When $A^{11}$ and $A^{12}$ each have an $R^N$, the two $R^N$ may be mutually the same or different.

In Formula (1), $B^{11}$ to $B^{18}$ each represents -N= or $-C(R^M)=$. Here, $R^M$ represents a hydrogen atom or a substituent.

The substituent that can be adopted as $R^M$ is not limited, and examples include groups selected from the substituent group Z above. The group selected from the substituent group Z above may further contain a substituent. Examples of such a substituent include a group selected from the substituent group Z. Examples of groups further containing a substituent include the combined group described above which may be adopted as $R^N$, and specific examples include the groups listed above, and further include groups having a methine group bonded to a carbon atom of the

compounds represented by Formula (1).

$R^M$ is preferably a hydrogen atom, an alkyl group, an alkenyl group, an alkoxycarbonyl group, an aryl group, an alkoxy group, a heterocyclic group (particularly a heteroaryl group), an amino group, a halogen atom, a cyano group, a carboxy group, a nitro group, or a mercapto group, more preferably a hydrogen atom, an alkyl group, an alkenyl group, an aryl group, an alkoxy group, a heterocyclic group (particularly a heteroaryl group), a halogen atom, or a cyano group, and particularly preferably a hydrogen atom, an alkyl group, an aryl group, a heterocyclic group (particularly a heteroaryl group), a halogen atom, or a cyano group.

Substituents that can be adopted as $R^M$ may form a ring. Aspects in which the substituents form a ring include an aspect in which the substituents bond to each other to form a ring, and an aspect in which a plurality of substituents share one atom to form a ring. Examples of the aspect in which the substituents bond to each other to form a ring include an aspect in which two vinyl groups bond to each other to form a benzene ring together with the carbon atom to which the $R^M$ bonds. Furthermore, examples of the aspect in which a plurality of substituents share one atom to form a ring include an aspect in which two substituents come together to form a sulfur atom (-S- group).

Among $B^{11}$ to $B^{18}$, preferably at least one is -N=, more preferably from 1 to 4 are -N=, even more preferably 1 or 2 are -N=, and particularly preferably two are - N=. Furthermore, an aspect in which all of $B^{11}$ to $B^{18}$ are -C($R^M$)= is also preferable.

The B which can be -N= is not limited, and any one of $B^{11}$ to $B^{18}$ may be -N=. For example, preferably, at least one of the group consisting of $B^{12}$, $B^{13}$, $B^{16}$, and $B^{17}$ is -N=, and more preferably, one or both of $B^{12}$ and $B^{16}$ is -N=.

-N=, which may be adopted as $B^{11}$ to $B^{18}$, may have a substituent on its nitrogen atom. Examples include an N-oxide group (N$\rightarrow$O group) and a salt having a counter anion.

In Formula (1), $X^{11}$ to $X^{14}$ each represent an oxygen atom or a sulfur atom, and preferably an oxygen atom. It is more preferable that all of $X^{11}$ to $X^{14}$ are oxygen atoms.

Here, the combination of $A^{11}$ and $A^{12}$ and $X^{11}$ to $X^{14}$ is not limited, but a combination in which $A^{11}$ and $A^{12}$ are -N($R^N$)- while $X^{11}$ to $X^{14}$ are oxygen atoms is preferable.

From the perspective of having high carrier mobility, the compound represented by Formula (1) is particularly preferably at least one compound selected from the group consisting of compounds represented by Formulas (1-1) to (1-5) below.

Compound represented by Formula (1-1): N,N'-bis(3-phenylpropyl)pyrene-3,4,9,10-dicarbodiimide
Compound represented by Formula (1-2): N,N'-bis(2-phenylethyl)pyrene-3,4,9,10-dicarbodiimide
Compound represented by Formula (1-3): N,N'-bis(octyl)pyrene-3,4,9,10-dicarbodiimide (C8PDI)
Compound represented by Formula (1-4): 2,9-bis(2,2,3,3,4,4,4-heptafluorobutyl)-1,2,3,8,9,10-hexahydro-1,3,8,10-tetraoxoanthra[2,1,9-def:6,5,10-d'e'f]diisoquinoline-5,12-dicarbonitrile (PDI-FCN2(1,7))
Compound represented by Formula (1-5): 2,9-bis[(1S)-1-methylpentyl]-1,2,3,8,9,10-hexahydro-1,3,8,10-tetraoxoanthra[2,1,9-def:6,5,10-d'e'f]diisoquinoline-5,12-dicarbonitrile (PDI1MPCN2 (1,7))

[Chem. 4]

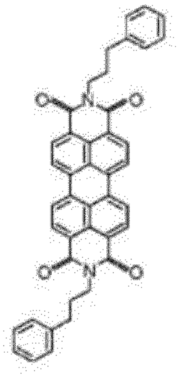

(1−1)

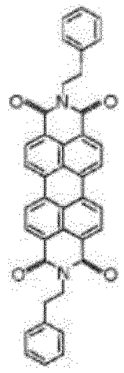

(1−2)

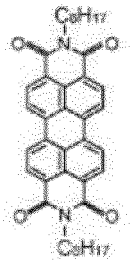

(1−3)

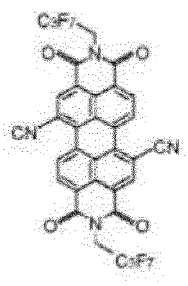

(1−4)

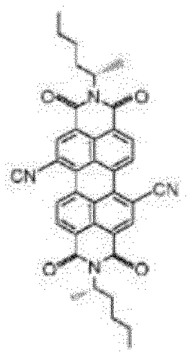

(1−5)

The compound represented by Formula (1) above can be produced by a production method described in, for example, WO 2011/082234 or JP 2018-006745 A, or a commercially available product can also be used.

The compound represented by Formula (1) above has a structure in which substituents are introduced at the imide nitrogen at both ends of the perylene diimide skeleton as the rigid main chain skeleton, and therefore the compound exhibits high solubility in 2,3-dihydrobenzofuran Compound (A), and does not easily precipitate even in low temperature environments.

As another example of an n-type organic semiconductor material as a solute contained in the ink composition according to an embodiment of the present disclosure, a compound having a naphthalene diimide skeleton as a rigid main chain skeleton is also preferable, and examples thereof include compounds represented by Formula (2) below and compounds represented by Formula (3) below:

[Chem. 5]

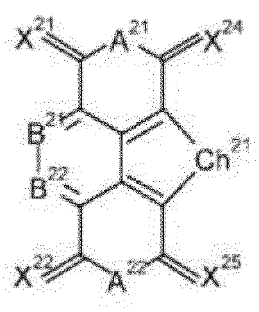

(2)

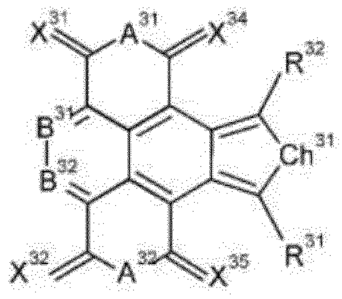

(3)

[In Formula (2), $A^{21}$ and $A^{22}$ each independently represent -N($R^{N1}$)-, -P($R^{N1}$)-, or -O-. $R^{N1}$ represents a hydrogen atom or a substituent. Each of the plurality of $R^{N1}$ may be the same or different.

In Formula (2), $B^{21}$ and $B^{22}$ each independently represent -N= or -C($R^{M1}$)=. $R^{M1}$ represents a hydrogen atom or a substituent. When $B^{21}$ and $B^{22}$ are both - C($R^{M1}$)=, the $R^{M1}$ contained in $B^{21}$ and the $R^{M1}$ contained in $B^{22}$ may form a ring.

In Formula (2), $Ch^{21}$ represents a sulfur atom, a sulfinyl group, a sulfonyl group, a selenium atom, a seleninyl group, a selenonyl group, or a group represented by -$B^{23}$-$B^{24}$-. $B^{23}$ and $B^{24}$ each independently represent -N= or -C($R^{M2}$)=. $R^{M2}$ represents a hydrogen atom or a substituent. When $B^{23}$ and $B^{24}$ are both -C($R^{M2}$)=, the $R^{M2}$ contained in $B^{23}$ and the $R^{M2}$ contained in $B^{24}$ may form a ring.

In Formula (2), $X^{21}$, $X^{22}$, $X^{24}$ and $X^{25}$ each independently represent an oxygen atom or a sulfur atom.

In Formula (3), $A^{31}$ and $A^{32}$ each independently represent -N($R^{N1}$)-, -P($R^{N1}$)-, or -O-. $R^{N1}$ represents a hydrogen atom or a substituent. Each of the plurality of $R^{N1}$ may be the same or different.

In Formula (3), $B^{31}$ and $B^{32}$ each independently represent -N= or -C($R^{M1}$)=. $R^{M1}$ represents a hydrogen atom or a substituent. When $B^{31}$ and $B^{32}$ are both - C($R^{M1}$)=, the $R^{M1}$ contained in $B^{31}$ and the $R^{M1}$ contained in $B^{32}$ may form a ring.

In Formula (3), $Ch^{31}$ represents a sulfur atom, a sulfinyl group, a sulfonyl group, a selenium atom, a seleninyl group, or a selenonyl group.

In Formula (3), $X^{31}$, $X^{32}$, $X^{34}$, and $X^{35}$ each independently represent an oxygen atom or a sulfur atom.

In Formula (3), $R^{31}$ and $R^{32}$ each independently represent a hydrogen atom or a substituent.]

In Formula (2), $A^{21}$ and $A^{22}$ each independently represent -N($R^{N1}$)-, -P($R^{N1}$)-, or -O-. Among these, from the perspective of further improving carrier mobility, $A^{21}$ and $A^{22}$ are each independently preferably -N($R^{N1}$)- or -P($R^{N1}$)-, and more preferably - N($R^{N1}$)-. $R^{N1}$ represents a hydrogen atom or a substituent. Each of the plurality of $R^{N1}$ may be the same or different. The substituent represented by $R^{N1}$ is not limited, and examples include a group selected from the substituent group Z described above. $A^{21}$ and $A^{22}$ are preferably the same group from the perspective of further improving carrier mobility.

Among these, $R^{N1}$ is preferably a hydrogen atom, a silyl group, a heterocyclic group, an aryl group, an alkynyl group, or a linear, branched, or cyclic alkyl group. Note that each of the groups mentioned above, not including the hydrogen atom, may be further substituted by a substituent selected from the substituent group Z described above. Among these, from the perspective of further improving carrier mobility, $R^{N1}$ is preferably a linear, branched or cyclic alkyl group having from 1 to 20 carbons, an aryl group having from 6 to 20 carbons, or a heteroaryl group having from 3 to 20 carbons, and is more preferably a linear, branched or cyclic alkyl group having from 1 to 20 carbons. Note that each of the groups mentioned above may be further substituted by a substituent selected from the substituent group Z described above. Furthermore, from the perspective of even further improving carrier mobility, $R^{N1}$ is even more preferably a cyclic alkyl group (cycloalkyl group) having from 3 to 8 carbons (preferably from 4 to 7 carbons, more preferably from 5 to 6 carbons), and is particularly preferably a cyclohexyl group.

In Formula (2), $B^{21}$ and $B^{22}$ each independently represent -N= or -C($R^{M1}$)=. From the perspective of improving air stability, $B^{21}$ and $B^{22}$ are preferably both - C($R^{M1}$)=, or one is -N= while the other is -C($R^{M1}$)=, and are more preferably both - C($R^{M1}$)=. When $B^{21}$ and $B^{22}$ are both -C($R^{M1}$)=, the $R^{M1}$ contained in $B^{21}$ and the $R^{M1}$ contained in $B^{22}$ may form a ring. When a ring is formed, an aromatic heterocyclic ring or an aromatic hydrocarbon ring is preferable, and a benzene ring is more preferable. Note that when $R^{M1}$ contained in $B^{21}$ and $R^{M1}$ contained in $B^{22}$ form a ring, the ring may have a substituent selected from the substituent group Z described above, or the substituents may bond together to further form a ring. $R^{M1}$ represents a hydrogen atom or a substituent. Note that in Formula (2), when there is a plurality of $R^{M1}$, each of the plurality of $R^{M1}$ may be the same or different. The substituent represented by $R^{M1}$ is not limited, and examples include a group selected from the substituent group Z described above. Among these, $R^{M1}$ is preferably a hydrogen atom, a halogen atom, an alkyl halide group, a cyano group, a nitro group, an alkoxy group, an alkoxycarbonyl group, a carboxy group, a heterocyclic group, or an amino group, more preferably a hydrogen atom, a halogen atom, or a cyano group, and even more preferably a hydrogen atom or a cyano group. In particular, when at least one of $B^{21}$ and $B^{22}$ in Formula (2) is -C($R^{M1}$)=, at least one $R^{M1}$ is preferably a halogen atom or a cyano group, and more preferably a cyano group. Through such a configuration, air stability is further improved.

In Formula (4), $Ch^{21}$ represents a sulfur atom, a sulfinyl group (-SO-), a sulfonyl group (-$SO_2$-), a selenium atom, a seleninyl group (-SeO-), a selenonyl group (-$SeO_2$-), or a group represented by -$B^{23}$-$B^{24}$-. $B^{23}$ and $B^{24}$ each independently represent -N= or -C($R^{M2}$)=. $R^{M2}$ represents a hydrogen atom or a substituent. When $B^{23}$ and $B^{24}$ are both -C($R^{M2}$)=, the $R^{M2}$ contained in $B^{23}$ and the $R^{M2}$ contained in $B^{24}$ may form a ring. From the perspective of further improving air stability, $B^{23}$ and $B^{24}$ are preferably both -C($R^{M2}$)=, or one is -N= while the other -C($R^{M2}$)=, and are more preferably both -C($R^{M2}$)=. When $B^{23}$ and $B^{24}$ are both -C($R^{M2}$)=, the $R^{M2}$ contained in $B^{23}$ and the $R^{M2}$ contained in $B^{24}$ may form a ring. When a ring is formed, an aromatic heterocyclic ring or an aromatic hydrocarbon ring is preferable, and a benzene ring is more preferable. Note that when $R^{M2}$ contained in $B^{23}$ and $R^{M2}$ contained in $B^{24}$ form a ring, the ring may have a substituent selected from the substituent group Z described above, or the substituents may bond together

to further form a ring. $R^{M2}$ represents a hydrogen atom or a substituent. Note that in Formula (2), when there is a plurality of $R^{M2}$, each of the plurality of $R^{M2}$ may be the same or different. The substituent group that can be adopted as $R^{M2}$ is not limited, and examples include groups selected from the substituent group Z described above. $Ch^{21}$ is preferably a sulfur atom, a selenium atom, or a group represented by $-B^{23}-B^{24}-$, from the perspective of further improving carrier mobility.

In Formula (2), $X^{21}$, $X^{22}$, $X^{24}$, and $X^{25}$ each independently represent an oxygen atom or a sulfur atom, but from the perspective of further improving air stability, $X^{21}$, $X^{22}$, $X^{24}$, and $X^{25}$ are preferably all oxygen atoms.

In Formula (3), $A^{31}$ and $A^{32}$ are synonymous with $A^{21}$ and $A^{22}$, respectively, in Formula (2), and each independently represents $-N(R^{N1})-$, $-P(R^{N1})-$, or -O-. $R^{N1}$ represents a hydrogen atom or a substituent. Each of the plurality of $R^{N1}$ may be the same or different. The substituent represented by $R^{N1}$ is not limited, and examples include a group selected from the substituent group Z described above. Furthermore, the preferred aspects of $A^{31}$ and $A^{32}$ in Formula (3) are also the same as the preferred aspects of $A^{21}$ and $A^{22}$ in Formula (2) above.

In Formula (3), $B^{31}$ and $B^{32}$ are synonymous with $B^{21}$ and $B^{22}$, respectively, in Formula (2), and each independently represents -N= or $-C(R^{M1})=$. $R^{M1}$ represents a hydrogen atom or a substituent. Note that in Formula (3), when there is a plurality of $R^{M1}$, each of the plurality of $R^{M1}$ may be the same or different. The substituent represented by $R^{M1}$ is not limited, and examples include a group selected from the substituent group Z described above. Furthermore, the preferred aspects of $B^{31}$ and $B^{32}$ in Formula (3) are also the same as the preferred aspects of $B^{21}$ and $B^{22}$ in Formula (2) above. When $B^{31}$ and $B^{32}$ are both $-C(R^{M1})=$, the $R^{M1}$ contained in $B^{31}$ and the $R^{M1}$ contained in $B^{32}$ may form a ring. The aspects in this case are also the same as in Formula (2) above.

In Formula (3), $X^{31}$, $X^{32}$, $X^{34}$, and $X^{35}$ are synonymous with $X^{21}$, $X^{22}$, $X^{24}$, and $X^{25}$, respectively, in Formula (2), and each independently represents an oxygen atom or a sulfur atom. Furthermore, the preferred aspects of $X^{31}$, $X^{32}$, $X^{34}$, and $X^{35}$ in Formula (3) are also the same as the preferred aspects of $X^{21}$, $X^{22}$, $X^{24}$, and $X^{25}$ in Formula (2) above.

In Formula (3), $Ch^{31}$ represents a sulfur atom, a sulfinyl group (-SO-), a sulfonyl group ($-SO_2-$), a selenium atom, a seleninyl group (-SeO-), or a selenonyl group ($-SeO_2-$), but is preferably a sulfur atom or a selenium atom from the perspective of further improving carrier mobility.

In Formula (3), $R^{31}$ and $R^{32}$ each independently represent a hydrogen atom or a substituent. The substituents represented by $R^{31}$ and $R^{32}$ are not particularly limited, and examples include a group selected from the substituent group Z described above. Among these, from the perspective of further improving carrier mobility, $R^{31}$ and $R^{32}$ are preferably each independently a hydrogen atom, a cyano group, a halogen atom, a silyl group, or a linear, branched or cyclic alkyl group having from 1 to 20 carbons, and more preferably a hydrogen atom, a methyl group, a halogen atom, or a cyano group.

The compounds represented by Formula (2) and the compounds represented by Formula (3) above can be produced by a production method described in, for example, WO 2011/082234 or WO 2017/022735, or a commercially available product can also be used.

The compound represented by Formula (2) above or the compound represented by Formula (3) above has a structure in which substituents are introduced at the imide nitrogen at both ends of the naphthalene diimide skeleton as the rigid main chain skeleton, and therefore, the compound is highly soluble in 2,3-dihydrobenzofuran Compound (A) and does not easily precipitate even at low temperatures.

Macromolecular compound

The ink composition according to an embodiment of the present disclosure may contain a macromolecular compound in addition to 2,3-dihydrobenzofuran Compound (A) and the solute described above. The macromolecular compound is preferably selected from inert polymers that do not affect the electrical properties of the organic semiconductor material, and examples include epoxy resins, acrylic resins, polystyrene resins, cellulose resins, and butyral resins; specifically, PMMA (polymethyl methacrylate), PS (polystyrene), PVA (polyvinyl alcohol), PVB (polyvinyl butyral), poly(2,3,4,5,6-pentafluorostyrene), PVP (polyvinyl phenol), BCB (benzocyclobutene), POSS (cage-like oligosilsesquioxane), PTFEMA (poly(2,2,2-trifluoroethyl methacrylate)), and P2VP (poly(2-vinylpyridine)) can be selected as appropriate.

In the edge-casting method or the continuous edge-casting method described below, in cases where an organic semiconductor material with low solubility is used, the inclusion of the above macromolecular compound has the effect of improving film formability, such as improving the in-plane uniformity of the organic single crystal. In addition, by containing the above macromolecular compound, the effect on the insulating film interface can be minimized, and high performance may be exhibited on any insulating film surface. The film formability referred to in the present specification means that during the forming of an organic single-crystal film, the film formability is good enough to form a film at a low temperature and a high speed at the indicated setting values of, for example, the ink composition temperature, the substrate temperature, the coating speed (single crystal growth speed), the slit temperature, the piping temperature, the ink tank temperature, and the distance between the slit and the substrate.

When the ink composition according to an embodiment of the present disclosure contains the macromolecular compound described above, the content of the macromolecular compound is not particularly limited, but is preferably from 0.01 to 20 wt.%, and more preferably from 0.1 to 10 wt.%, per 100 wt.% of the ink composition. When the content of the macromolecular compound is within this range, the film formability of the ink composition according to an embodiment of the present disclosure tends to be improved.

The ink composition according to an embodiment of the present disclosure contains 2,3-dihydrobenzofuran Compound (A) as a solvent, a solute (in particular, an organic semiconductor material), and, as necessary, the aforementioned macromolecular compound. A single type of each of 2,3-dihydrobenzofuran Compound (A), the solute, and the macromolecular compound to be combined as necessary can be used, or a combination of two or more type of each can be used.

The ink composition according to an embodiment of the present disclosure can be prepared, for example, by mixing 2,3-dihydrobenzofuran Compound (A), the solute, and the macromolecular compound to be combined as necessary, and heating the mixture at a temperature of around from 30 to 100°C in an air atmosphere, a nitrogen atmosphere, or an argon atmosphere for approximately from 0.1 to 5 hours.

The content of 2,3-dihydrobenzofuran Compound (A) (the total amount if two or more types are contained) in the total amount of the ink composition according to an embodiment of the present disclosure is, for example, not greater than 99.999 wt.%. The lower limit is, for example, 90.000 wt.%, preferably 93.000 wt.%, particularly preferably 95.000 wt.%, while the upper limit is preferably 99.990 wt.%.

The content of the solute (in particular, the organic semiconductor material) (the total amount if two or more types are contained) in the ink composition according to an embodiment of the present disclosure is, for example, 0.02 parts by weight or greater, preferably 0.03 parts by weight or greater, and particularly preferably 0.04 parts by weight or greater, per 100 parts by weight of 2,3-dihydrobenzofuran Compound (A). The upper limit of the content of the solute is, for example, 1 part by weight, preferably 0.5 parts by weight, and particularly preferably 0.1 parts by weight.

The weight ratio (solute/polymeric compound) of the solute (in particular, the organic semiconductor material) to the macromolecular compound in the ink composition according to an embodiment of the present disclosure is, for example, 0.01 or greater, preferably 0.02 or greater, and particularly preferably 0.1 or greater. The upper limit of the weight ratio is, for example, 1000, preferably 500, more preferably 100, even more preferably 50, and particularly preferably 10.

Organic thin-film transistor

Next, a structure of an organic thin-film transistor that is provided with an organic single-crystal semiconductor and in which is used the ink composition for manufacturing an organic semiconductor device according to an embodiment of the present disclosure, and a method for manufacturing an organic thin-film transistor, the method including applying, onto a substrate, the ink composition for manufacturing an organic semiconductor device according to an embodiment of the present disclosure, and forming an organic single-crystal semiconductor film are described with reference to FIGS. 1 and 2.

FIG. 1 is a schematic diagram of a cross-sectional structure of an example of the organic thin-film transistor. The organic thin-film transistor includes a flexible resin substrate 101, a conductive thin film (gate electrode) 102, a gate insulating film 103, an organic single-crystal semiconductor thin film 104, a conductive thin film (source electrode, drain electrode) 105, a charge injection layer 106 for ohmic contact formation, and a protective layer 107, which are formed on a temporary fixing substrate 100 (also referred to as a carrier substrate) for handling during processing. The production of the organic thin-film transistor is described briefly below.

First, the conductive thin film 102 is formed on the resin substrate 101, which is temporarily fixed on the temporary fixing substrate 100 for handling, and is used as a gate electrode for the organic thin-film transistor.

Examples of the method of forming the conductive thin film 102 include a PVD method exemplified by sputtering and a vacuum deposition method, or a method in which the conductive thin film 102 is formed on the resin substrate 101 by a coating method using an ink containing a conductive material and then patterned into a predetermined shape by photolithography.

Other methods of forming the conductive thin film 102 include, for example, a method of directly forming, on the resin substrate 101, a conductive thin film 102 that has been patterned into a predetermined shape, using a plate-based printing method or a non-plate printing method. By directly forming the conductive thin film 102 that has been patterned into a predetermined shape, the process can be simplified.

In addition, the conductive thin film 102 may be formed by a plating method. An example of the method of forming the conductive thin film 102 by a plating method includes a method in which first, a plating primer layer patterned into a predetermined shape in advance is formed on the resin substrate 101 by photolithography, a plate-based printing method, or a non-plate printing method, and then the conductive thin film 102 is formed at a predetermined position by an electroless plating method, or a combination of an electroless plating method and an electrolytic plating method.

The thickness of the conductive thin film 102 is not limited, but is preferably from 20 nm to 1 μm, and more preferably from 20 nm to 300 nm.

Next, the gate insulating film 103 is formed on the resin substrate 101 and the conductive thin film 102. The gate insulating film 103 is preferably an organic insulating film containing a macromolecular compound or a ferroelectric such as a ceramic represented by a metal compound having high relative permittivity. The thickness of the gate insulating film 103 is not limited, but is preferably from 1 nm to 1 μm, more preferably from 10 nm to 600 nm, and even more preferably from 10 nm to 200 nm.

Next, an organic single-crystal semiconductor film 104' not patterned into the shape of a transistor is formed on the gate insulating film 103 using the ink composition according to an embodiment of the present disclosure by a drop-casting method, an inkjet printing method, an edge-casting method, or a continuous edge-casting method. The edge-casting method or the continuous edge- casting method can be performed in accordance with a known method (for example, the method described in JP 2015-185620 A).

FIG. 2 is a conceptual view of an example of a method for forming the organic single-crystal semiconductor film by a continuous edge-casting method. As a brief explanation of FIG. 2, at least a substrate stage 200 for placing a substrate on a continuous edge-casting apparatus, a slit 201 for continuous edge-casting application and ink supply, and an ink tank 202 are provided, ink is supplied to the substrate surface by pressurizing the ink tank 202, and an ink meniscus 203 is formed, after which the organic single-crystal semiconductor film 104' is obtained by fine-tuning all of the parameters such as the application speed (single crystal growth speed), heating of each part of the apparatus, heating of the substrate, and the evaporation rate. The drop-casting and inkjet printing methods can also be performed in accordance with known methods.

The organic single-crystal semiconductor film 104' that is formed by a drop-casting method, an inkjet printing method, an edge-casting method, or a continuous edge-casting method but is not yet patterned into the shape of a transistor is subsequently patterned into a predetermined shape by photolithography, and thereby the organic single-crystal semiconductor thin film 104 is formed.

Furthermore, after forming the organic single-crystal semiconductor thin film 104, a firing treatment may be performed to control the morphology or volatilize the solvent contained in the organic single-crystal semiconductor thin film 104. The thickness of the organic single-crystal semiconductor thin film 104 is not limited, but is preferably from 1 nm to 1000 nm, more preferably from 1 nm to 100 nm, and even more preferably from 1 nm to 50 nm.

The best film is more preferably a crystal film that is not thick and has from 3 to 6 molecular layers or less, and the optimum total number of molecular layers varies depending on the molecular structure.

Next, the conductive thin film 105 that has been patterned is formed on the gate insulating film 103 and the organic single-crystal semiconductor thin film 104. The conductive thin film 105 forms the source electrode and the drain electrode of the organic thin-film transistor.

The conductive thin film 105 may be formed by the same methods as those of forming the conductive thin film 102 described above. Note that the methods of forming the conductive thin film 105 and the conductive thin film 102 may be the same or different. Additionally, as necessary, the charge injection layer 106 for causing ohmic contact between the organic single-crystal semiconductor thin film 104 and the conductive thin film 105 may be provided between the organic single-crystal semiconductor thin film 104 and the conductive thin film 105.

The film thickness of the conductive thin film 105 (that is, the film thickness of the source electrode and the drain electrode of the organic thin-film transistor) is not particularly limited, but is preferably from 20 nm to 1 μm, more preferably from 20 nm to 600 nm, and even more preferably from 20 nm to 500 nm.

Next, the protective layer 107 is formed on the gate insulating film 103, the organic single-crystal semiconductor thin film 104, and the conductive thin film 105. Examples of the method of forming the protective layer 107 include a PVD method exemplified by a vacuum deposition method, a CVD method exemplified by an atomic layer deposition (ALD) method, and a method in which the protective layer 107 is formed by a coating method using an ink containing a protective layer material and then patterned into a predetermined shape by photolithography.

Other methods of forming the protective layer 107 include, for example, a method of directly forming, through a plate-based printing method or a non-plate printing method, the protective layer 107 that has been patterned into a predetermined shape. By directly forming the protective layer 107 that has been patterned into a predetermined shape, the process of forming the protective layer 107 can be simplified.

Of these, the method of directly forming, through a plate-based printing method or a non-plate printing method, the protective layer 107 that has been patterned into a predetermined shape is preferable. As yet another method, patterning may be performed by forming holes in predetermined places by laser ablation.

When the protective layer 107 that has been patterned into a predetermined shape is directly formed through a plate-based printing method or a non-plate printing method, an ink containing various protective layer materials can be used. Examples of the ink containing protective layer materials include a dispersed ink containing an inorganic material, a SOG (spin-on glass) material, an ink containing a low-molecular protective layer material, and an ink containing a macromolecular protective layer material, of which an ink containing a macromolecular protective layer material is

preferable.

Examples of the material that forms the protective film 107 include the materials contained in the ink described above, the SOG materials, as well as the same materials as those exemplified in the description regarding the gate insulating film 103 above.

The film thickness of the protective layer 107 is not particularly limited, but is preferably from 50 nm to 5.0 μm, and more preferably from 500 nm to 3.0 μm.

Finally, the flexible resin substrate 101 is peeled from the temporary fixing substrate 100, thereby completing the organic thin-film transistor on a flexible substrate. A laser lift-off method (LLO) can be used as the method for peeling off the flexible resin substrate 101. Alternatively, it is possible to form, in advance, a release layer or a weak adhesive layer between the temporary fixing substrate 100 and the flexible resin substrate 101 using, for example, a fluorine-based polymer, a self-assembled monolayer (SAMs), or a week adhesive, and then physically peel off the resin substrate 101 after completion. Of course, it is possible to first form the release layer or weak adhesive layer and then peel off the resin substrate 101 using LLO. In this way, the organic thin-film transistor can be produced.

Because the ink composition according to an embodiment of the present disclosure uses 2,3-dihydrobenzofuran Compound (A) as a solvent, a high concentration of a solute (in particular, an organic semiconductor material) can be dissolved even at a relatively low temperature (for example, from 20 to 50°C, and preferably from 20 to 40°C). Furthermore, high film formability and advanced printing properties can be exhibited in the above-described temperature environment because the ink composition has appropriate volatility suited for the printing process and the concentration of the ink composition is kept constant. Therefore, even in a low temperature environment, an organic semiconductor device can be easily formed by a simple method using a wet process such as the edge-casting method and the continuous edge-casting method described above, and costs can be significantly reduced.

In addition, the organic semiconductor device can be formed directly on a plastic substrate, which is lower in heat resistance than a glass substrate but is flexible, lightweight and strongly impact-resistant, and thus displays and computer equipment that are impact-resistant, lightweight, and flexible can be formed. Furthermore, when the ink composition according to an embodiment of the present disclosure is applied to a substrate, the solute (n-type organic semiconductor material) contained in the composition crystallizes through a self-assembly action, and an organic semiconductor device (for example, an organic thin-film transistor) having high carrier mobility is obtained.

The transistor characteristics of the organic thin-film transistor produced by a method according to an embodiment of the present disclosure can be measured, for example, through the carrier mobility as indicated below.

Evaluation of Carrier Mobility

Carrier mobility can be evaluated, for example, using the test conditions of 1 or 2 below.

Test condition 1: Using a semiconductor parameter analyzer (4200-SCS, available from Keithley), a voltage of +50 V is applied between the source electrode and the drain electrode of the organic thin-film transistor at an ordinary atmospheric pressure of 1 atm (temperature: room temperature), the gate voltage is changed in a range of from -20 V to +50 V, and the following equation representing the drain current $I_d$ is used to calculate the carrier mobility μ ($cm^2/Vs$).

Test condition 2: Using a semiconductor parameter analyzer (4200-SCS, available from Keithley), a voltage of +40 V is applied between the source electrode and the drain electrode of the organic thin-film transistor at an ordinary atmospheric pressure of 1 atm (temperature: room temperature), the gate voltage is changed in a range of from -20 V to +40 V, and the following equation representing the drain current $I_d$ is used to calculate the carrier mobility μ ($cm^2/Vs$).

$$I_d = (w/2L)\mu C_i(V_g - V_{th})^2$$

In the above equation, L is the gate length, w is the gate width, μ is the carrier mobility, $C_i$ is the capacitance per unit area of the gate insulating film, $V_g$ is the gate voltage, and $V_{th}$ is the threshold voltage.

The carrier mobility μ of the organic thin film transistor manufactured by a method according to an embodiment of the present disclosure is preferably high, and is for example, 0.1 $cm^2/Vs$ of greater, preferably 0.3 $cm^2/Vs$ or greater, more preferably 0.5 $cm^2/Vs$ or greater, even more preferably 0.7 $cm^2/Vs$ or greater, and particularly preferably 1.0 $cm^2/Vs$ or greater.

As examples of applications of the organic thin-film transistor described above, the organic thin-film transistor can be used, without any particular limitations, in electronic paper, display devices, sensors, and electronic tags.

The various configurations, combinations thereof, and the like of each embodiment described above are merely examples, and various configurational additions, omissions, substitutions, and other changes may be made as appropriate within a range that does not depart from the spirit of invention according to the present disclosure. The invention according to the present disclosure is not limited by the embodiments and is limited only by the scope of the claims.

Each aspect disclosed in the present specification can be combined with any other feature disclosed herein.

Examples

Hereinafter, the invention according to the present disclosure will be described more specifically through examples, but the invention according to the present disclosure is not limited by these examples.

Example 1: 2,9-bis[(1S)-1-methylpentyl]-1,2,3,8,9,10-hexahydro-1,3,8,10-tetraoxoanthra[2,1,9-def:6,5,10-d'e'f']diisoquinoline-5,12-dicarbonitrile (a compound represented by Formula (1-5), hereinafter referred to as compound (1-5)) was mixed as a solute in 2,3-dihydrobenzofuran (available from Daicel Corporation) as a solvent with the amount of the compound (1-5) being 0.05 wt.%, the mixture was heated for 3 hours at 80°C to dissolve the compound (1-5), and a solution of an ink composition for manufacturing an organic semiconductor device was obtained.

The obtained solution was coated onto a fluorine-based resin substrate using the continuous edge-casting method illustrated in FIG. 2, and a single-crystal film was produced. An organic thin-film transistor illustrated in FIG. 1 was fabricated on the produced single-crystal film at 25 locations. The transistor characteristics of the obtained organic thin-film transistor were measured under the above-described test condition 1 for evaluating carrier mobility. A carrier mobility performance of 0.66 $cm^2/Vs$ was obtained as an average of the 25 locations.

Example 2: 0.1 wt.% of the compound (1-5) was mixed as a solute in 2,3-dihydrobenzofuran (available from Daicel Corporation) as a solvent, and 0.04 wt.% of poly-$\alpha$-methylstyrene was mixed therein as an additive, the mixture was heated for 3 hours at 80°C to dissolve the materials, and a solution of an ink composition for manufacturing an organic semiconductor device was obtained. The weight ratio (solute/macromolecular compound) of the solute (compound (1-5)) to the macromolecular compound (poly-$\alpha$-methylstyrene) was 2.5. The obtained solution was coated onto a parylene-coated resin substrate using the continuous edge-casting method illustrated in FIG. 2, and a single-crystal film was produced. An organic thin-film transistor illustrated in FIG. 1 was fabricated on the produced single-crystal film at 25 locations. The transistor characteristics of the obtained organic thin-film transistor were measured under the above-described test condition 1 for evaluating carrier mobility. A carrier mobility performance of 0.32 $cm^2/Vs$ was obtained as an average of 19 locations.

Example 3: 0.1 wt.% of the compound (1-5) was mixed as a solute in 2,3-dihydrobenzofuran (available from Daicel Corporation) as a solvent, and 0.04 wt.% of poly-$\alpha$-methylstyrene was mixed therein as an additive, the mixture was heated for 3 hours at 80°C to dissolve the materials, and a solution of an ink composition for manufacturing an organic semiconductor device was obtained. The weight ratio (solute/macromolecular compound) of the solute (compound (1-5)) to the macromolecular compound (poly-$\alpha$-methylstyrene) was 2.5. The obtained solution was used to form a self-assembled monolayer on the resin substrate. The obtained solution was also coated onto the self-assembled monolayer using the continuous edge-casting method illustrated in FIG. 2, and a single-crystal film was produced. An organic thin-film transistor illustrated in FIG. 1 was fabricated on the produced single-crystal film at 5 locations. The transistor characteristics of the obtained organic thin-film transistor were measured under the above-described test condition 2 for evaluating carrier mobility. A carrier mobility performance of 0.76 $cm^2/Vs$ was obtained as an average of the 5 locations.

To summarize the above, configurations and variations of the invention according to the present disclosure are described below.

[1] An ink composition containing at least one solvent selected from 2,3-dihydrobenzofuran Compound (A) represented by Formula (a), and at least one type of solute.

[2] The ink composition according to [1], wherein $R^1$, $R^2$, $R^3$, $R^4$, $R^5$ and $R^6$ in Formula (a) each represent a hydrogen atom, a halogen atom or a $C_{1-20}$ alkyl group optionally having a substituent selected from Group 1 described above.

[3] The ink composition according to [2], wherein the halogen atom is a fluorine atom, a chlorine atom, a bromine atom, or an iodine atom.

[4] The ink composition according to [2] or [3], wherein the $C_{1-20}$ alkyl group is a linear or branched-chain alkyl group having from 1 to 20 carbons.

[5] The ink composition according to [1], wherein 2,3-dihydrobenzofuran Compound (A) is 2,3-dihydrobenzofuran and/or 2,3-dihydro-2-methylbenzofuran.

[6] The ink composition according to [1], wherein 2,3-dihydrobenzofuran Compound (A) is 2,3-dihydrobenzofuran.

[7] The ink composition according to any of [1] to [6], wherein a proportion of 2,3-dihydrobenzofuran Compound (A) in a total of the solvent is greater than or equal to 90 wt.% (preferably greater than or equal to 93 wt.%, more preferably greater than or equal to 95 wt.%, and even more preferably 100 wt.%).

[8] The ink composition according to any of [1] to [7], wherein a purity of 2,3-dihydrobenzofuran Compound (A) is 98.0% or greater (preferably 98.5% or greater, more preferably 99.0% or greater, even more preferably 99.5% or greater, and particularly preferably 99.9% or greater).

[9] The ink composition according to any of [1] to [8], wherein a water content of the solvent is not greater than 0.25 wt.% (preferably not greater than 0.19 wt.%, and more preferably not greater than 0.05 wt.%).

[10] The ink composition according to any of [1] to [9], wherein the solute is an n-type organic semiconductor material.

[11] The ink composition according to [10], wherein the n-type organic semiconductor material is a compound represented by Formula (1).

[12] The ink composition according to [11], wherein both $A^{11}$ and $A^{12}$ in Formula (1) are $-N(R^N)-$.

[13] The ink composition according to [12], wherein the $R^N$ is an alkyl group that has from 4 to 20 carbons and may have a substituent.

[14] The ink composition according to [13], wherein the substituent is an unsubstituted alkyl group, an alkyl halide group, or an aryl group.

[15] The ink composition according to any of [11] to [14], wherein $B^{11}$ to $B^{18}$ in Formula (1) are -N= or $-C(R^M)-$.

[16] The ink composition according to any of [11] to [15], wherein $X^{11}$ to $X^{14}$ in Formula (1) are oxygen atoms.

[17] The ink composition according to any of [11] to [14], wherein $X^{11}$ to $X^{14}$ in Formula (1) are oxygen atoms, and $B^{11}$ to $B^{18}$ are $-C(R^M)-$.

[18] The ink composition according to any of [15] to [17], wherein the $R^M$ is a hydrogen atom, an alkyl group, an allyl group, a heteroaryl group, a halogen group, or a cyano group.

[19] The ink composition according to [11], wherein the compound represented by Formula (1) is at least one compound selected from the group consisting of compounds represented by Formula (1-1) to Formula (1-5).

[20] The ink composition according to [11], wherein the compound represented by Formula (1) is a compound represented by Formula (1-5).

[21] The ink composition according to [10], wherein the n-type organic semiconductor material is a compound represented by Formula (2).

[22] The ink composition according to [21], wherein both $A^{21}$ and $A^{22}$ in Formula (2) are $-N(R^{N1})-$.

[23] The ink composition according to [22], wherein $R^{N1}$ is a cyclohexyl group.

[24] The ink composition according to any of [21] to [23], wherein $B^{21}$ and $B^{22}$ in Formula (2) are $-C(R^{M1})=$.

[25] The ink composition according to [24], wherein $R^{M1}$ is a halogen atom or a cyano group, and one or both $R^{M1}$ are a cyano group.

[26] The ink composition according to any of [21] to [25], wherein $Ch^{21}$ in Formula (2) is $-B^{23}-B^{24}-$.

[27] The ink composition according to [26], wherein $B^{23}$ and $B^{24}$ are - $C(R^{M}2)=$.

[28] The ink composition according to [27], wherein $R^{M2}$ is a halogen atom or a cyano group.

[29] The ink composition according to any of [21] to [28], wherein $X^{21}$, $X^{22}$, $X^{24}$, and $X^{25}$ in Formula (2) are oxygen atoms.

[30] The ink composition according to [10], wherein the n-type organic semiconductor material is a compound represented by Formula (3).

[31] The ink composition according to [30], wherein both $A^{31}$ and $A^{32}$ in Formula (3) are $-N(R^{N1})-$.

[32] The ink composition according to [31], wherein $R^{N1}$ is a cyclohexyl group.

[33] The ink composition according to any of [30] to [32], wherein $B^{31}$ and $B^{32}$ in Formula (3) are $-C(R^{M1})=$.

[34] The ink composition according to [33], wherein $R^{M1}$ is a halogen atom or a cyano group, and one or both $R^{M1}$ are a cyano group.

[35] The ink composition according to any of [30] to [34], wherein $X^{31}$, $X^{32}$, $X^{34}$, and $X^{35}$ in Formula (3) are oxygen atoms.

[36] The ink composition according to any of [30] to [35], wherein $Ch^{31}$ in Formula (3) is a sulfur atom or a selenium atom.

[37] The ink composition according to any of [30] to [36], wherein $R^{31}$ and $X^{32}$ in Formula (3) are each a hydrogen atom, methyl group, halogen atom, or cyano group.

[38] The ink composition according to any of [1] to [37], further including a macromolecular compound as a second component in addition to the solute.

[39] The ink composition according to [38], wherein the macromolecular compound is a polystyrene resin.

[40] The ink composition according to [39], wherein the polystyrene resin is poly -$\alpha$-methyl styrene.

[41] The ink composition according to any of [1] to [40], which is used for producing an organic single-crystal semiconductor film by a drop-casting method, an inkjet printing method, an edge-casting method, or a continuous edge-casting method.

[42] The ink composition according to any of [1] to [40], which is used for producing an organic single-crystal semiconductor film by a continuous edge-casting method.

[43] A method of manufacturing an organic single-crystal semiconductor film using the ink composition described in any one of [1] to [42].

[44] The manufacturing method according to [43], wherein a film thickness of the organic single-crystal semiconductor film is from 1 nm to 100 nm.

[45] The manufacturing method according to [43], wherein the organic single-crystal semiconductor film is a crystalline film having six molecular layers or less.

[46] Use of the ink composition described in any one of [1] to [42] as an ink for manufacturing an organic semiconductor

device.

[47] A method for producing an ink composition, wherein the ink composition described in any one of [1] to [42] is produced by mixing at least 2,3-dihydrobenzofuran Compound (A) and a solute, and heating at 30 to 100°C for about 0.1 to 5 hours.

[48] A method for producing an ink for manufacturing an organic semiconductor device, wherein the ink composition described in [42] is an ink for manufacturing an organic semiconductor device.

Industrial Applicability

A high-performance organic thin-film transistor can be efficiently obtained at a low cost by manufacturing an n-type organic single-crystal transistor using the ink composition for manufacturing an organic semiconductor device according to an embodiment of the present disclosure. In particular, by using the ink composition in combination with a drop-casting method, an inkjet printing method, an edge-casting method, or a continuous edge-casting method, the performance of an n-type organic semiconductor material can be maximally exhibited.

Reference Signs List

100 Temporary fixing substrate for handling
101 Resin substrate
102 Conductive thin film (gate electrode)
103 Gate insulating film
104 Organic semiconductor thin film
105 Conductive thin film (source electrode, drain electrode)
106 Charge injection layer for ohmic contact formation
107 Protective layer
200 Substrate stage for placing a substrate on a continuous edge-casting apparatus
201 Slit for continuous edge-casting application and ink supply
202 Ink tank
203 Meniscus of an ink composition for manufacturing an organic device, the meniscus being formed between a slit and a substrate

## Claims

**1.** An ink composition for manufacturing an organic semiconductor device, the ink composition comprising at least one solvent selected from 2,3-dihydrobenzofuran Compound (A) described below, and at least one type of solute:

2,3-dihydrobenzofuran Compound (A), which includes compounds represented by Formula (a):

[Chem. 1]

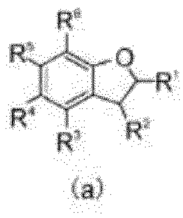

(a)

where, in Formula (a), $R^1$, $R^2$, $R^3$, $R^4$, $R^5$, and $R^6$ each represent a hydrogen atom, a halogen atom, a $C_{1-20}$ alkyl group optionally having a substituent selected from Group 1, a $C_{2-22}$ alkenyl group optionally having a substituent selected from Group 1, a $C_{2-22}$ alkynyl group optionally having a substituent selected from Group 1, a $C_{1-20}$ alkoxy group optionally having a substituent selected from Group 1, a $C_{1-20}$ alkylthio group optionally having a substituent selected from Group 1, a $C_{2-20}$ alkylcarbonyl group optionally having a substituent selected from Group 1, a $C_{2-20}$ alkoxycarbonyl group optionally having a substituent selected from Group 1, a di- or mono- $C_{1-20}$ alkylamino group optionally having a substituent selected from Group 1, a $C_{6-20}$ aryl group optionally

having a substituent selected from Group 2, a monovalent heterocyclic group optionally having a substituent selected from Group 2, or a $C_{3-20}$ cycloalkyl group optionally having a substituent selected from Group 2, wherein Group 1 includes a halogen atom, a sulfonyl group, a hydroxy group, an aldehyde group (-CHO), a carbonyl group, a carboxyl group, a nitro group, an amino group, a sulfo group ($-SO_3H$), an ether group, a $C_{1-20}$ alkylthio group, di- or mono- $C_{1-20}$ alkylamino group, a $C_{6-20}$ aryl group, a monovalent heterocyclic group, and a $C_{3-20}$ substituted silyl group; and

Group 2 includes a substituent selected from Group 1, a $C_{1-20}$ alkyl group optionally having a substituent selected from Group 1, a $C_{2-20}$ alkenyl group optionally having a substituent selected from Group 1, and a $C_{2-20}$ alkynyl group optionally having a substituent selected from Group 1.

**2.** The ink composition for manufacturing an organic semiconductor device according to claim 1, wherein $R^1$, $R^2$, $R^3$, $R^4$, $R^5$ and $R^6$ each represent a hydrogen atom, a halogen atom or a $C_{1-20}$ alkyl group optionally having a substituent selected from Group 1.

**3.** The ink composition for manufacturing an organic semiconductor device according to claim 1 or 2, wherein 2,3-dihydrobenzofuran Compound (A) is 2,3-dihydrobenzofuran.

**4.** The ink composition for manufacturing an organic semiconductor device according to any one of claims 1 to 3, wherein the solute is an n-type organic semiconductor material.

**5.** The ink composition for manufacturing an organic semiconductor device according to any one of claims 1 to 4, the ink composition further comprising a macromolecular compound as a second component in addition to the solute.

**6.** The ink composition for manufacturing an organic semiconductor device according to any one of claims 1 to 5, which is used for producing an organic single-crystal semiconductor film by a drop-casting method, an inkjet printing method, an edge-casting method, or a continuous edge-casting method.

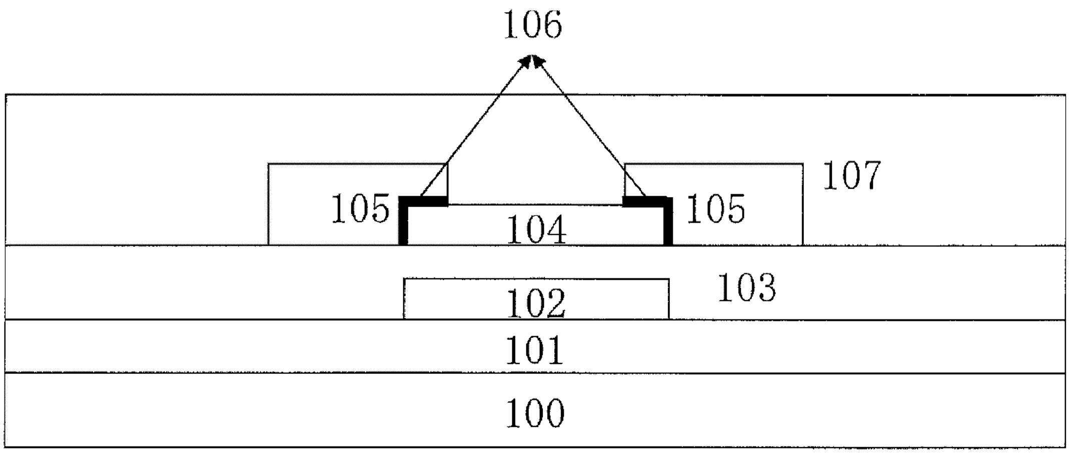


FIG. 1

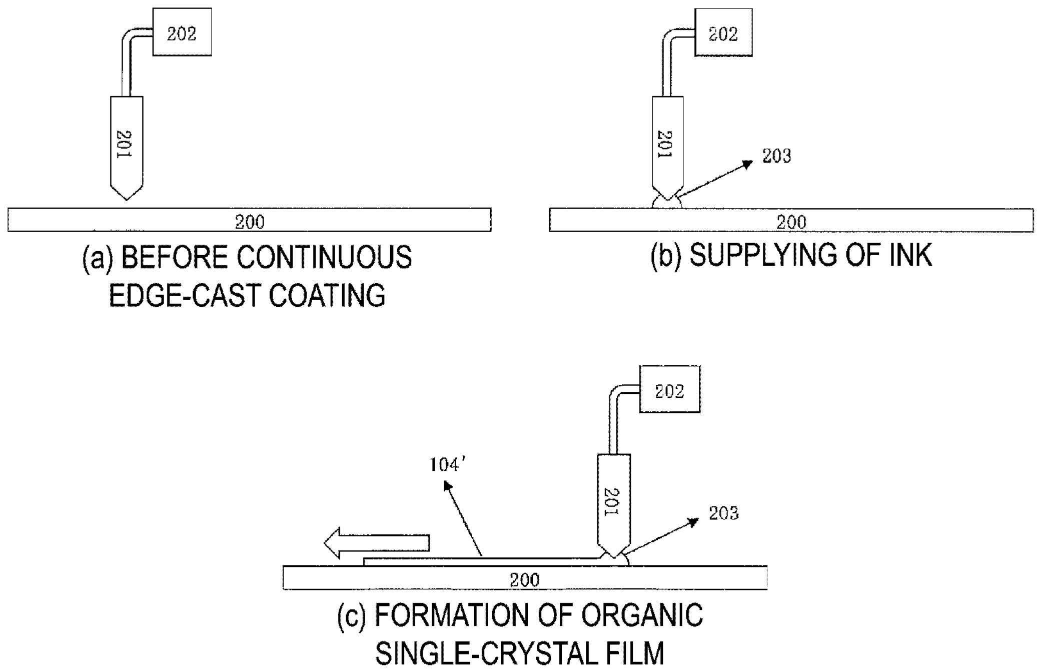
202
201
200
(a) BEFORE CONTINUOUS
EDGE-CAST COATING
202
201
203
200
(b) SUPPLYING OF INK
202
104'
201
203
200
(c) FORMATION OF ORGANIC
SINGLE-CRYSTAL FILM

FIG. 2

## INTERNATIONAL SEARCH REPORT

International application No.
PCT/JP2020/015555

A. CLASSIFICATION OF SUBJECT MATTER
H01L 51/30(2006.01)i; C07D 307/79(2006.01)i; C07D 471/04(2006.01)i; H01L 21/330(2006.01)i; H01L 29/780(2006.01)i; H01L 51/05(2006.01)i; H01L 51/40(2006.01)i
FI: H01L29/28 250H; H01L29/28 310J; H01L29/28 100A.; H01L29/78 618B; H01L29/78 618A.; C07D307/79; C07D471/04 112Z
According to International Patent Classification (IPC) or to both national classification and IPC

B. FIELDS SEARCHED
Minimum documentation searched (classification system followed by classification symbols)
C07D471/04; H01L51/05; H01L51/30; H01L51/40; H01L21/336; H01L29/786; C07D307/79

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2017-532768 A (BASF SE) 02.11.2017 (2017-11-02) paragraphs [0018], [0088], [0188], [0198], [0209], [0210] | 1-6<br>6 |
| X<br>Y | JP 2015-195301 A (FUJIFILM CORPORATION) 05.11.2015 (2015-11-05) paragraphs [0043], [0070], [0085], [0088] | 1-6<br>6 |
| X<br>Y | WO 2017/212882 A1 (DAICEL CORPORATION) 14.12.2017 (2017-12-14) paragraphs [0042], [0045], [0051] | 1-3, 5, 6<br>6 |
| Y | JP 2017-528915 A (BASF SE) 28.09.2017 (2017-09-28) paragraph [0018], fig. 1 | 6 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 June 2020 (16.06.2020) | 30 June 2020 (30.06.2020) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**
Information on patent family members

International application no.
PCT/JP2020/015555

| Patent Documents referred in the Report | Publication Date | Patent Family | Publication Date |
|---|---|---|---|
| JP 2017-532768 A | 02 Nov. 2017 | US 2017/0250347 A1 paragraphs [0047], [0136], [0463], [0500], [0511], [0512]<br>WO 2016/027217 A1<br>KR 10-2017-0042358 A<br>CN 107004768 A | |
| JP 2015-195361 A | 05 Nov. 2015 | US 2017/0012220 A1 paragraphs [0159], [0160], [0238], [0293], [0304]<br>TW 201546078 A<br>CN 106165105 A<br>KR 10-2016-0127074 A | |
| WO 2017/212882 A1 | 14 Dec. 2017 | US 2019/0214580 A1 paragraphs [0044], [0047], [0053]<br>CN 109314186 A<br>KR 10-2019-0016964 A<br>TW 201819389 A | |
| JP 2017-528915 A | 28 Sep. 2017 | US 2017/0244045 A1 paragraph [0021], fig. 1<br>TW 201618346 A<br>KR 10-2017-0045320 A<br>CN 107207492 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2019077362 A
- JP 2015185620 A
- JP 2018006745 A
- WO 2011082234 A
- WO 2017022735 A